# FASCICULE DE BREVET EUROPEEN

(11) **EP 1 086 945 B1**
(45) Date de publication et mention de la délivrance du brevet: **09.10.2002**
(21) Numéro de dépôt: 00402369.3
(22) Date de dépôt: 28.08.2000
(51) Int. Cl.: C07C 233/58, A61K 7/48, A61K 47/48

(54) **Composition comprenant un composé dérivé de cyclohexane, composé et utilisation dudit composé pour structurer une composition**
Zusammensetzung enthaltend ein Cyclohexanderivat, Verbindung und deren Verwendung zur Strukturierung einer Zusammensetzung
Composition containing a cyclohexane derivative, compound and use thereof for structuring the composition

(30) Priorité: 21.09.1999 FR 9911773
(43) Date de publication de la demande: 28.03.2001
(73) Titulaire: L'OREAL, 75008 Paris (FR)
(72) Inventeur: Livoreil, Aude, 75006 Paris (FR)
(74) Mandataire: Dodin, Catherine

(56) Documents cités:
- EP-A- 0 291 334
- PATENT ABSTRACTS OF JAPAN vol. 1998, no. 13, 30 novembre 1998 (1998-11-30) & JP 10 212213 A (POLA CHEM IND INC), 11 août 1998 (1998-08-11)
- PATENT ABSTRACTS OF JAPAN vol. 1999, no. 01, 29 janvier 1999 (1999-01-29) & JP 10 273477 A (HANABUSA KENJI;POLA CHEM IND INC), 13 octobre 1998 (1998-10-13)
- PATENT ABSTRACTS OF JAPAN vol. 1998, no. 14, 31 décembre 1998 (1998-12-31) & JP 10 237034 A (POLA CHEM IND INC), 8 septembre 1998 (1998-09-08)

## Description

La présente invention a trait à une composition notamment solide notamment cosmétique, telle qu'une composition de soin, de traitement et/ou de maquillage de la peau, y compris du cuir chevelu, et/ou des lèvres des êtres humains, ladite composition comprenant une phase grasse liquide épaissie, et se présentant notamment sous forme d'un stick ou bâton de maquillage comme un rouge à lèvres.

Dans les compositions notamment cosmétiques et dermatologiques, il est courant d'utiliser une phase grasse liquide structurée, c'est-à-dire épaissie ou gélifiée, pour obtenir la consistance souhaitée. L'épaississement des huiles (ou des phases liquides à température ambiante) permet en particulier de faciliter la prise du produit hors de son conditionnement sans perte significative, de limiter la diffusion du produit à la zone locale de traitement, de répartir le produit de façon régulière sur la zone locale de traitement ou bien encore de pouvoir utiliser le produit dans des quantités suffisantes pour obtenir l'effet cosmétique ou dermatologique recherché Ceci est notamment le cas dans les compositions solides comme les déodorants, les baumes et les rouges à lèvres, les produits anti-cerne et les fonds de teint coulés. Cet épaississement est notamment primordial pour les compositions de soin, d'hygiène ou de maquillage comme les rouges à lèvres qui doivent bien se répartir de façon homogène sur la surface locale à traiter ainsi que pour les compositions capillaires qui doivent s'étaler et se répartir de façon régulière le long des fibres kératiniques et ne pas ruisseler sur le front, la nuque, le visage ou dans les yeux.
Pour remédier à ces problèmes, on a habituellement recourt à des cires ou des charges. Malheureusement, ces cires et/ou charges ont tendance à matifier la composition et à la rendre opaque, ce qui n'est pas toujours souhaitable en particulier pour un rouge à lèvres. En effet, les femmes sont toujours à la recherche d'un rouge à lèvres sous forme d'un bâton permettant l'obtention d'un film brillant; par ailleurs, certaines compositions telles que les baumes à lèvres ou les onguents, peuvent se présenter sous forme de sticks translucides, voire transparents.
Il est également connu d'épaissir les huiles avec des épaississants polymériques. Malheureusement, les épaississants d'huiles connus doivent être utilisés en grande quantité pour obtenir un gel de viscosité élevée, par exemple supérieure à 1,3 Pa.s. Or, une trop grande quantité d'épaississant peut conférer à la composition des propriétés cosmétiques inadéquates, notamment un toucher collant et un manque de glissant, ces inconvénients pouvant être très gênants, voire rédhibitoires.
Par ailleurs, il est également connu de gélifier des compositions, notamment cosmétiques, en utilisant un gélifiant de type tri-alkyl,tri-(alkylaminocarbonyl)-cyclohexane. Ces gélifiants permettent d'améliorer la stabilité des compositions les comprenant. Toutefois, les gels obtenus sont, une fois encore, peu transparents.

La structuration de la phase grasse liquide permet en particulier de limiter son exsudation des compositions solides et, en plus, de limiter, après dépôt sur la peau ou les lèvres, la migration de cette phase dans les rides et ridules, ce qui est particulièrement recherché pour un rouge à lèvres. En effet, une migration importante de la phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

La présente invention a pour but de proposer l'obtention d'une composition notamment cosmétique, pouvant se présenter sous forme solide, et présentant une bonne translucidité, voire transparence.

L'invention a donc pour objet une composition notamment cosmétique ou dermatologique, pouvant se présenter sous forme solide, comprenant au moins un composé défini par la formule (I) suivante : 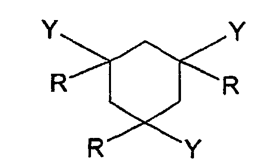 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
   - un atome d'hydrogène,
   - un groupement aryle, éventuellement substitué par une chaîne hydrocarbonée saturée ou insaturée, linéaire ou ramifiée, comprenant 1 à 22 atomes de carbone, notamment 10-18 atomes de carbone; ou
   - une chaîne hydrocarbonée saturée ou insaturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy;
à la condition qu'au moins un desdits radicaux R' comprenne au moins une chaîne hydrocarbonée insaturée.

L'invention a également pour objet un procédé de traitement cosmétique d'un support choisi parmi la peau du visage ou du corps, les muqueuses et les fibres kératiniques, comprenant l'application sur ledit support d'une composition telle que ci-dessus définie.
Un autre objet de l'invention est l'utilisation, dans une composition cosmétique ou dermatologique se présentant sous forme solide et comprenant au moins une huile, d'une quantité suffisante d'au moins un composé de formule (I) pour structurer/gélifier ladite composition.

Notamment, la composition peut se présenter sous la forme d'un stick anhydre translucide voire transparent. Elle trouve une application particulière comme composition "sans transfert" ou "non migrante" éventuellement colorée.

On a en effet constaté que l'utilisation des composés de formule (I) permet de structurer et d'épaissir fortement les phases grasses liquides (ou huileuses), voire de les gélifier complètement, et ainsi d'obtenir des compositions cosmétiques stables sous forme gélifiée solide, présentant des propriétés cosmétiques satisfaisantes. Ces compositions peuvent même être exemptes de cires tout en conservant leur rigidité et leurs bonnes propriétés cosmétiques.
La composition selon l'invention présente de bonnes propriétés cosmétiques : elle n'est pas collante lors de l'application et est glissante et facile à appliquer. Elle permet l'obtention d'un film homogène et uniforme, couvrant et confortable à porter.
De plus, la composition peut avantageusement être claire, transparente ou translucide.
On entend par là la définition classique donnée dans le dictionnaire. Ainsi, une composition translucide laisse passer la lumière sans permettre toutefois de distinguer nettement les contours des objets. Une composition transparente se laisse aisément traverser par la lumière et permet de distinguer nettement les objets à travers son épaisseur.
D'une manière générale, une composition transparente aura une valeur de transmittance maximum de la lumière, quelque soit la longueur d'onde comprise entre 400 et 800 nm, à travers un échantillon de 1 cm d'épaisseur, d'au moins 35%, de préférence d'au moins 50% (voir EP291334).
Une composition translucide aura, quant à elle, une valeur de transmittance maximum de la lumière comprise entre 2 et 35%.
La transmittance peut être mesurée en plaçant un échantillon de 1 cm d'épaisseur dans le rayon lumineux d'un spectrophotomètre travaillant dans les longueurs d'onde du spectre lumineux.
Il a également été constaté que, de manière surprenante et inattendue, les compositions selon l'invention permettaient l'obtention d'un dépôt pouvant être très brillant et très lisse.

Par ailleurs, les composés de formule (I) peuvent avantageusement être utilisés pour préparer des compositions "sans transfert" notamment colorées, pour lesquelles la migration du film coloré dans les rides et ridules, notamment autour des lèvres ou des yeux est très limitée. Ces compositions présentent également l'avantage de ne pas, ou peu, se déposer sur certains supports avec lesquels elles sont mises en contact, tels que, par exemple, un verre, un vêtement ou la peau.

La composition selon l'invention comprend donc au moins un composé correspondant à la formule (I): 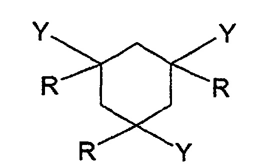 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
   - un atome d'hydrogène,
   - un groupement aryle, éventuellement substitué par une chaîne hydrocarbonée saturée ou insaturée, linéaire ou ramifiée, comprenant 1 à 22 atomes de carbone, notamment 10-18 atomes de carbone; ou
   - une chaîne hydrocarbonée saturée ou insaturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy;
à la condition qu'au moins un desdits radicaux R' comprenne au moins une chaîne hydrocarbonée insaturée.

Par chaîne hydrocarbonée insaturée, on entend une chaîne qui comprend au moins une double liaison C=C, ou au moins une triple liaison C≡C, ladite chaîne pouvant bien entendu éventuellement en outre être substituée par un ou plusieurs groupements choisis parmi les groupements aryle, ester, amide, uréthanne; et/ou comprendre éventuellement un ou plusieurs hétéroatomes choisi parmi O, S et N; et/ou éventuellement être substituée par un ou plusieurs atomes de fluor et/ou radicaux hydroxy.
Au moins un des radicaux R' comprend donc au moins une chaîne hydrocarbonée insaturée. Cette chaîne hydrocarbonée insaturée peut donc soit représenter directement ledit radical R', soit peut être reliée à un groupement aryle; dans ce dernier cas, R' représente donc un groupement aryle substitué par ladite chaîne hydrocarbonée insaturée.
Il est également possible d'avoir un mélange de ces deux possibilités.

De préférence, au moins un des radicaux R', mieux au moins deux, et encore mieux les trois radicaux R', représente(nt) une chaîne hydrocarbonée comportant une seule double insaturation, linéaire ou ramifiée, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy.

Encore plus préférentiellement, les radicaux R' représentent une chaîne hydrocarbonée comportant une seule double insaturation, linéaire ou ramifiée, comprenant 1 à 22 atomes de carbone, notamment 10-18 atomes de carbone. On peut en particulier citer les radicaux caproléyle, lauroléyle, myristoléyle, palmitoléyle, oléyle, gadoléyle, linoléyle, linolényle et élaïdyle.

Dans un mode de réalisation préféré, les trois radicaux R' sont identiques et sont choisis dans la liste ci-dessus.

De préférence, R représente un atome d'hydrogène ou un radical méthyle.

De préférence, Y représente un groupement -CO-NHR' ou -NH-COR'.
Les trois substituants représentés par Y peuvent être, dans le composé de formule I, en conformation cis-cis, cis-trans ou trans-trans, les uns par rapport aux autres.
Notamment, au moins un de ces substituants peut être placé en position équatoriale sur le cycle cyclohexane; de préférence, tous les substituants Y sont placés en position équatoriale.

Parmi les composés susceptibles d'être employés dans le cadre de l'invention, on peut citer :
- le cis-1,3,5-tris(oléylaminocarbonyl)cyclohexane,
- le cis-1,3,5-tris(palmitoylaminocarbonyl)cyclohexane,
- le cis-1,3,5-tris(lauroylaminocarbonyl)cyclohexane,
- le cis-1,3,5-tris(gadoleylaminocarbonyl)cyclohexane,
- le cis-1,3,5-tris(élaïdylaminocarbonyl)cyclohexane,
- le cis-1,3-bis(oléylaminocarbonyl)-cis-5-(octadécylaminocarbonyl)cyclohexane,
- le cis-1,3-bis(oléylaminocarbonyl)-cis-5-(dodécylaminocarbonyl)cyclohexane,
- le cis-1,3-bis(oléylaminocarbonyl)-cis-5-[N-(3,7-diméthyloctyl)-aminocarbonyl] cyclohexane,
- le cis-1-(oléylaminocarbonyl)-cis-3,5-bis(octadécylaminocarbonyl)cyclohexane,
- le cis-1-(oléylaminocarbonyl)-cis-3,5-bis(dodécylaminocarbonyl)cyclohexane,
- le cis-1-(oléylaminocarbonyl)-cis-3,5-bis[N-(3,7-diméthyloctyl)-aminocarbonyl] cyclohexane,
- le trans-1,3,5-triméthyl-1,3,5-tris(oléylaminocarbonyl)cyclohexane, et
- le trans-1,3,5-triméthyl-1,3,5-tris(gadoléylaminocarbonyl)cyclohexane.

Les composés de formule (I) peuvent être préparés selon des procédés bien connus de l'homme du métier.
Ces composés étant nouveaux, ils forment également un objet de l'invention.
Ils sont de préférence présents dans la composition en une quantité aisément déterminable par l'homme du métier en fonction de l'effet recherché, et qui peut être comprise entre 1 et 40% en poids, par exemple 2-10% en poids par rapport au poids total de la composition, et encore mieux 3-8% en poids, voire 4-6% en poids.
On a par ailleurs constaté que même l'utilisation d'une faible quantité de composés de formule (I), par exemple de l'ordre de 2-6% en poids, pouvait conduire à une gélification adéquate de la composition selon l'invention. Ceci est dû à un fort pouvoir épaississant des composés de formule (I), qui leur permet d'être efficace à faible concentration, de l'ordre de 2-6% en poids, alors qu'il serait nécessaire d'utiliser 10-20% en poids de gélifiants usuels pour obtenir un résultat équivalent.
Sans être tenu par la présente explication, on a constaté que la structuration, ou gélification, des huiles grâce aux composés de formule (I) pouvait être due à la formation d'amoncellements sous forme de colonnes des molécules de composés de formule (I), d'où la constitution d'un réseau de fibres ou feuillets, constitué par lesdits composés de formule (I) et par les huiles, ledit réseau ne diffractant pas la lumière, d'où une certaine translucidité, voire transparence.

Les composés de formule (I) peuvent notamment être employés, seul ou en mélange, dans une composition qui comprend un milieu physiologiquement acceptable, notamment dans une composition cosmétique qui comprend donc par ailleurs un milieu cosmétiquement acceptable.
Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

Généralement, mais non nécessairement, la composition selon l'invention va comprendre au moins une huile, liquide à température ambiante (25°C) cosmétiquement ou dermatologiquement acceptable.
Ces huiles peuvent être des huiles hydrocarbonées et/ou siliconées et/ou fluorées. Elles peuvent être d'origine animale, végétale, minérale ou synthétique.

On peut en particulier citer :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque; les huiles de tournesol, de maïs, de soja, de courge, de pépins de raisin, d'arachide, d'amande douce, de calophyllum, de palme, de sésame, de noisette, d'abricot, de macadamia, de ricin, d'avocat; les triglycérides des acides caprylique/caprique comme ceux vendus par la société Stearineries Dubois ou ceux vendus sous les dénominations Miglyol 810, 812 et 818 par la société Dynamit Nobel; l'huile de jojoba, de beurre de karité ;
- les hydrocarbures linéaires ou ramifiés, d'origine minérale ou synthétique tels que les huiles de paraffine et leurs dérivés, la vaseline, les polydécènes, l'huile de Purcellin, le polyisobutène hydrogéné tel que le parléam ;
- les esters et les éthers de synthèse notamment d'acides gras comme les huiles de formule R₃COOR₄ dans laquelle R₃ représente le reste d'un acide gras supérieur comportant de 7 à 29 atomes de carbone et R₄ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées;
- les huiles siliconées comme les polyméthylsiloxanes (PDMS) volatiles ou non, linéaires ou cycliques; les alkyldiméthicones; les silicones modifiées par des groupements aliphatiques et/ou aromatiques, éventuellement fluorés, ou par des groupements fonctionnels tels que des groupements hydroxyles, thiols et/ou amines; les huiles siliconées phénylées telles que les polyphénylméthylsiloxanes ou les phényltriméthicones.
- leurs mélanges.

Les huiles employées peuvent être volatiles et/ou non volatiles. Par huile volatile, on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de 10⁻³ à 300 mm de Hg (0,13 Pa à 40.000 Pa).
On peut notamment citer les huiles siliconées volatiles, telles que les silicones volatiles cycliques ou linéaires, et les cyclocopolymères. On peut également citer les huiles volatiles hydrocarbonées telles que les isoparaffines, et les huiles fluorées volatiles.
Dans un mode de réalisation particulier, les huiles volatiles peuvent constituer la majeure partie de la phase huileuse. Ainsi, elles peuvent y être présentes à raison de au moins 50% en poids, notamment au moins 75% en poids, voire 100% en poids, de ladite phase huileuse.

Les huiles peuvent être présentes dans la composition à raison de 5 à 99% en poids du poids total de la composition, de préférence de 20 à 75% en poids.
La composition selon l'invention se présente préférentiellement sous forme solide. On entend par là qu'on n'observe aucun affaissement de la composition en dehors du récipient la comprenant, en l'absence de stimulation mécanique ou thermique (chauffage notamment).
La composition présente un comportement viscoélastique classique d'un comportement de type solide.
Par ailleurs, la dureté de la composition selon l'invention est de préférence telle que la composition est autoportée et peut se déliter aisément pour former un dépôt satisfaisant sur la peau et les lèvres. Cette dureté peut être comprise entre 0,04 N et 3 N, de préférence entre 0,1 et 2,5 N, notamment entre 0,5 et 2N. Cette dureté peut être mesurée selon une méthode de pénétration d'une sonde dans ladite composition et en particulier à l'aide d'un analyseur de texture (par exemple-TA-XT2 de chez Rhéo) équipé d'un cône en acrylique d'angle au sommet de 45°. La mesure de dureté est effectuée à 22°C au centre de 5 échantillons de ladite composition selon la méthode décrite dans les exemples.

Ainsi, de manière avantageuse, cette composition peut comprendre peu (moins de environ 5% en poids par rapport au poids total de la composition), voire pas, de cire, tout en conservant une solidité/rigidité/dureté adéquate.
De préférence, la composition comprend moins de 2% en poids, voire moins de 0,5% en poids de cire. Préférentiellement, la composition ne contient pas de cires (soit 0%).
Une cire, au sens de la présente invention, est un composé gras lipophile, solide à température ambiante (environ 25°C), à changement d'état solide/liquide réversible, ayant une température de fusion supérieure à environ 40°C pouvant aller jusqu'à 200°C, et présentant à l'état solide une organisation cristalline anisotrope.
Les cires généralement utilisées dans les domaines cosmétique et dermatologique, sont notamment naturelles d'origine animale, végétale ou minérale, comme la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège.
On peut également citer les cires de paraffine, les cires microcristallines, la cire de lanoline, les ozokérites, les huiles hydrogénées ayant une température de fusion supérieure à environ 40°C comme l'huile de jojoba hydrogénée, les cires de polyéthylène issues de la polymérisation de l'éthylène, les cires obtenues par synthèse de Fischer-Tropsch, les esters d'acide gras et les glycérides ayant une température de fusion supérieure à environ 40°C, les cires de silicone comme les alkyle, alcoxy et/ou esters de poly(di)méthylsiloxane solide à 40°C.

La composition selon l'invention peut comprendre par ailleurs les constituants usuellement utilisés dans le type d'application envisagé.
Elle peut comprendre un ou plusieurs solvants organiques, notamment choisis parmi:
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol ;
- les éthers de propylène glycol liquides à température ambiante tels que le monométhyléther de propylène glycol, l'acétate de monométhyléther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde.

Il est en outre possible d'incorporer dans la composition selon l'invention une phase hydrophile, notamment en une quantité de 0-10% en poids par rapport au poids total de la composition, et mieux de 1-5% en poids, pouvant comprendre des actifs hydrophiles et/ou des gélifiants hydrophiles. Elle peut notamment comprendre des hydratants tels que la glycérine.
Avantageusement, la composition comprend une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40% du poids total de la composition, de préférence de 5 à 25% en poids.

Ainsi, la composition peut comprendre une phase particulaire, généralement présente à raison de 0-30% en poids, de préférence 0-20% en poids, et qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être blancs ou colorés, minéraux et/ou organiques, de taille micrométrique ou nanométrique. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Parmi les nacres envisageables, on peut citer le mica recouvert d'oxyde de titane, d'oxyde de fer, de pigment naturel ou d'oxychlorure de bismuth ainsi que le mica titane coloré.
Les charges peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et les microbilles de résine de silicone (Tospearls de Toshiba, par exemple).

La composition selon l'invention peut comprendre en outre tout additif usuellement utilisé dans le domaine considéré, notamment le domaine cosmétique, tel que des antioxydants, des parfums, des colorants, des huiles essentielles, des conservateurs, des actifs cosmétiques, des vitamines, des acides gras essentiels, des sphingolipides, des composés auto-bronzants tels que la DHA, des filtres solaires, des tensioactifs, des gélifiants, des polymères notamment hydrocarbonés, tels que le polybutène, les polyalkylènes, les polyacrylates et les polymères ou dérivés siliconés compatibles avec les corps gras. Ces additifs peuvent être présents dans la composition à raison de 0-10% en poids.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention sont destinées à être appliquées sur la peau du visage et du corps, sur les muqueuses et/ou sur les fibres kératiniques telles que les ongles, les cils ou les cheveux.
Elles peuvent se présenter sous toutes les formes galéniques envisageables, telles que gel huileux, comprenant éventuellement de l'eau, solide ou souple; émulsion solide ou gélifiée, huile-dans-eau, eau-dans-huile ou multiple; dispersion d'huile dans l'eau; système multiphases notamment biphase. Elles peuvent avoir l'aspect d'une crème, d'une pommade, d'une pâte souple, d'un onguent, d'un solide coulé ou moulé et notamment d'un stick.
Elles peuvent notamment se présenter sous forme de stick ou de coupelle; et en particulier sous forme d'un gel rigide anhydre transparent, et plus spécialement sous forme de stick anhydre translucide ou transparent.
La gélification de l'huile est telle que l'on peut obtenir une structure rigide sous forme d'un bâton ou d'un stick. Ces bâtons lorsqu'ils sont colorés permettent, après application, d'obtenir un dépôt homogène en couleur et ne migrant pas dans les rides et ridules de la peau, entourant en particulier les lèvres, mais aussi les yeux.

Ces compositions trouvent notamment une application comme composition d'hygiène corporelle, par exemple sous forme de sticks déodorants; comme composition capillaire, par exemple comme stick de coiffage ou stick de maquillage des cheveux; comme composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; comme composition de soin de la peau ou des muqueuses, par exemple comme baume ou base de soin pour les lèvres, onguent pour le corps, crème de soin journalier; comme composition solaire ou auto-bronzante.

Ces compositions trouvent une application toute particulière comme composition de maquillage ou de soin non transfert, notamment comme rouge à lèvre non transfert ou fond de teint non transfert.

L'invention est illustrée plus en détail dans les exemples suivants.

### Exemple 1 : Préparation du cis-1,3,5-tris(oléylaminocarbonyl)cyclohexane

On dissout 2 g d'acide cis-1,3,5-cyclohexane-tricarboxylique dans 50 ml de chloroforme. On ajoute 6 g de chlorure de thionyle, on mélange pendant 1 heure à température ambiante (25°C) et on concentre le mélange obtenu.
On ajoute 3 g d'oléylamine dissous dans 50 ml de chlorure de méthylène, et 10 ml de triéthylamine. On agite en chauffant le mélange à 50°C pendant 2 heures.
On récupère le précipitat, on le lave à l'eau, et l'on obtient 5 g de composé recherché.

### Exemple 2

Le composé utilisé dans cet exemple correspond à la formule (I) dans laquelle les trois radicaux R représentent l'hydrogène et les trois radicaux Y représentent le groupement -CO-NHR' avec R' représentant une chaîne hydrocarbonée linéaire ayant 18 atomes de carbone, comportant une double liaison (chaîne oléyle) :

CH₃-(CH₂)₇-CH=CH-(CH₂)₈-

A/
   On mélange 50 mg de ce composé avec 5 ml d'octyldodécanol, sous agitation à température ambiante, soit un mélange à 1% en composé (I). Le mélange est chauffé à 107°C sous agitation, jusqu'à homogénéisation. Il devient alors transparent, homogène et fluide. On laisse alors le mélange homogène refroidir lentement jusqu'à température ambiante.
   On obtient alors une composition transparente, solide et dure, qui ne s'affaisse pas en dehors du récipient, en l'absence de toute stimulation mécanique ou thermique. Cette composition peut être étalée par simple pression et permet l'obtention d'un film huileux et homogène.
B/
   On mesure la dureté du stick obtenu à l'aide d'un analyseur de texture TA-XT2 (société Rhéo), à 22°C, en utilisant un cône lisse en acrylique, d'angle au sommet 45°, et de hauteur totale supérieure à la distance de pénétration. Le cône pénètre à l'intérieur de l'échantillon d'une distance de 5 mm, à une vitesse de 2 mm/s. Il est ensuite maintenu immobile pendant 300 s, puis retiré de l'échantillon à une vitesse de 2 mm/s. La force exercée par l'échantillon sur le corps de mesure est enregistrée en continu. La force maximale est détectée à la fin de la phase de pénétration. Cette valeur de force reflète la dureté de l'échantillon.

On obtient le résultat suivant : 0,042 N

### Exemple 3 (exemple comparatif)

Pour comparaison, on prépare selon l'exemple 1, un mélange de 5 ml d'octyldodécanol et de 50 mg de composé correspondant à la formule (I) dans laquelle les trois radicaux R représentent l'hydrogène et les trois radicaux Y représentent le groupement -CO-NHR' avec R' représentant une chaîne hydrocarbonée saturée linéaire ayant 18 atomes de carbone (radical stéaryle).

On obtient alors une composition faiblement translucide, solide et dure.

Par ailleurs, on note qu'il est nécessaire de chauffer le mélange jusqu'à une température d'au moins 120°C pour obtenir un mélange homogène.

### Exemple 4: mesures de transparence

La mesure de la transparence ou de la translucidité est effectuée par mesure de la transmittance, soit le pourcentage de lumière transmise à travers un échantillon donné, dans le domaine des longueurs d'onde correspondant au domaine visible, soit entre 400 et 800 nm.
Cette transmittance est mesurée en continu au travers d'un échantillon d'huile épaissie, placé dans une cuve de verre de chemin optique 1 cm, par différence avec un échantillon dit de référence contenant la même huile pure.
L'instrument de mesure est un spectrophotomètre PERKIN-ELMER Lambda UV-Vis.

La composition est chauffée jusqu'à ce qu'elle soit sous forme d'un fluide homogène et est versée directement dans la cuve de mesure. La cuve est maintenue à température ambiante jusqu'au refroidissement de son contenu. On place ensuite la cuve dans l'appareil, la cuve de référence contenant de l'octyldodécanol pur étant placée dans l'appareil également.
On mesure la transmittance entre 400 et 800 nm.

On obtient les résultats suivants :
- composition de l'exemple 2 : la transmittance varie de manière continue quasi-linéaire, de 89% à 400 nm à 96% à 800 nm (valeur maximale).
   Ceci correspond bien à une composition transparente.
- composition-témoin de l'exemple 3 : la transmittance varie de manière continue quasi-linéaire, de 2% à 400 nm à 16% à 800 nm (valeur maximale).
   Ceci correspond bien à une composition très faiblement translucide.

### Exemple 5

On mélange 250 mg du composé de l'exemple 2 avec 5 ml d'isododécane et 25 mg de pigment (oxydes de fer), sous agitation à température ambiante. Le mélange est chauffé à 107°C jusqu'à homogénéisation. Il devient transparent, coloré, homogène et fluide. On laisse alors le mélange refroidir lentement jusqu'à température ambiante.
On obtient alors une composition solide et colorée, sous la forme d'un stick. Cette composition ne montre pas de séparation du pigment dans le temps. Elle permet l'obtention d'un film huileux et homogène.

### Exemple 6

De manière similaire aux exemples précédents, on prépare une composition selon l'invention comprenant :
- composé de l'exemple 2 0,8 g
- pigments (oxydes de fer) 0,5 g
- isododécane 16 ml
- huile de parléam 4 ml

On obtient un stick solide dur et coloré.

A l'aide de la composition ainsi préparée, on dépose un film coloré sur une plaque de verre. On laisse le dépôt sécher pendant 20 minutes. Le dépôt est alors sec mais reste malléable.
On applique un mouchoir en papier sur le dépôt, et on presse manuellement. On ne constate aucune trace colorée sur le mouchoir.
Le frottement mécanique du mouchoir sur le dépôt n'entraîne aucun transfert de couleur (entraînement de matière éventuellement).
La composition ainsi préparée présente bien de bonnes propriétés de non-transfert.

## Revendications

1. Composition notamment cosmétique ou dermatologique, pouvant se présenter sous forme solide, comprenant au moins un composé défini par la formule (I) suivante : 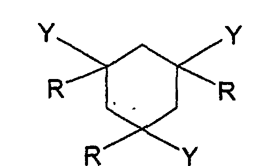 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
- un atome d'hydrogène,
- un groupement aryle, éventuellement substitué par une chaîne hydrocarbonée saturée ou insaturée, linéaire ou ramifiée, comprenant 1 à 22 atomes de carbone, notamment 10-18 atomes de carbone; ou
- une chaîne hydrocarbonée saturée ou insaturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy;
à la condition qu'au moins un desdits radicaux R' comprenne au moins une chaîne hydrocarbonée insaturée.

2. Composition selon la revendication 1, dans laquelle au moins un des radicaux R', de préférence au moins deux, et encore mieux les trois radicaux R', du composé de formule (I), représente(nt) une chaîne hydrocarbonée comportant une seule double insaturation, linéaire ou ramifiée, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy.

3. Composition selon l'une des revendications précédentes, dans laquelle les radicaux R' du composé de formule (I) représentent une chaîne hydrocarbonée comportant une seule double insaturation, linéaire ou ramifiée, comprenant 1 à 22 atomes de carbone, notamment 10-18 atomes de carbone.

4. Composition selon l'une des revendications précédentes, dans laquelle les radicaux R' du composé de formule (I) sont choisis parmi les radicaux caproléyle, lauroléyle, myristoléyle, palmitoléyle, oléyle, gadoléyle, linoléyle, linolényle et élaïdyle.

5. Composition selon l'une des revendications précédentes, dans laquelle, dans la formule (I):
- R représente un atome d'hydrogène ou un radical méthyle, et/ou
- Y représente un groupement -CO-NHR' ou -NH-COR'.

6. Composition selon l'une des revendications précédentes, dans laquelle le composé de formule (I) est choisi parmi :
- le cis-1,3,5-tris(oléylaminocarbonyl)cyclohexane,
- le cis-1,3,5-tris(palmitoylaminocarbonyl)cyclohexane,
- le cis-1,3,5-tris(lauroylaminocarbonyl)cyclohexane,
- le cis-1,3,5-tris(gadoleylaminocarbonyl)cyclohexane,
- le cis-1,3,5-tris(élaïdylaminocarbonyl)cyclohexane,
- le cis-1,3-bis(oléylaminocarbonyl)-cis-5-(octadécylaminocarbonyl)cyclohexane,
- le cis-1,3-bis(oléylaminocarbonyl)-cis-5-(dodécylaminocarbonyl)cyclohexane,
- le cis-1,3-bis(oléylaminocarbonyl)-cis-5-[N-(3,7-diméthyloctyl)-aminocarbonyl] cyclohexane,
- le cis-1-(oléylaminocarbonyl)-cis-3,5-bis(octadécylaminocarbonyl)cyclohexane,
- le cis-1-(oléylaminocarbonyl)-cis-3,5-bis(dodécylaminocarbonyl)cyclohexane,
- le cis-1-(oléylaminocarbonyl)-cis-3,5-bis[N-(3,7-diméthyloctyl)-aminocarbonyl] cyclohexane,
- le trans-1,3,5-triméthyl-1,3,5-tris(oléylaminocarbonyl)cyclohexane, et
- le trans-1,3,5-triméthyl-1,3,5-tris(gadoléylaminocarbonyl)cyclohexane.

7. Composition selon l'une des revendications précédentes, dans laquelle le composé de formule (I) est présent en une quantité comprise entre 1 et 40% en poids, par exemple 2-10% en poids par rapport au poids total de la composition, et encore mieux 3-8% en poids, voire 4-6% en poids.

8. Composition selon l'une des revendications précédentes, comprenant par ailleurs au moins une huile cosmétiquement ou dermatologiquement acceptable.

9. Composition selon l'une des revendications précédentes, comprenant par ailleurs au moins une huile choisie parmi les huiles hydrocarbonées et/ou siliconées et/ou fluorées, d'origine animale, végétale, minérale ou synthétique, volatile ou non volatile.

10. Composition selon l'une des revendications précédentes, comprenant moins de environ 5% en poids par rapport au poids total de la composition de cire, notamment moins de 2% en poids, voire moins de 0,5% en poids de cire, de préférence pas de cires (soit 0%).

11. Composition selon l'une des revendications précédentes, se présentant sous forme solide.

12. Composition selon l'une des revendications précédentes, ayant une dureté comprise entre 0,04 N et 3 N, de préférence entre 0,1 et 2,5 N, notamment entre 0,5 et 2N.

13. Composition selon l'une des revendications précédentes, se présentant sous forme translucide voire transparente.

14. Composition selon l'une des revendications précédentes, ayant une valeur de transmittance maximum de la lumière, quelque soit la longueur d'onde comprise entre 400 et 800 nm, à travers un échantillon de 1 cm d'épaisseur, d'au moins 2%.

15. Composition selon l'une des revendications précédentes, se présentant sous la forme d'une composition destinée à être appliquées sur la peau du visage et du corps, sur les muqueuses et/ou sur les fibres kératiniques telles que les ongles, les cils ou les cheveux.

16. Composition selon l'une des revendications précédentes, se présentant sous la forme d'un gel huileux, comprenant éventuellement de l'eau, solide ou souple; d'une émulsion solide ou gélifiée, huile-dans-eau, eau-dans-huile ou multiple; d'une dispersion d'huile dans l'eau; d'un système multiphases notamment biphase; d'une crème, d'une pommade, d'une pâte souple, d'un onguent, d'un solide coulé ou moulé et notamment d'un stick; d'un gel rigide anhydre transparent, et plus spécialement sous forme de stick anhydre translucide ou transparent.

17. Composition selon l'une des revendications précédentes, se présentant sous la forme d'une composition d'hygiène corporelle, par exemple sous forme de sticks déodorants; comme composition capillaire, par exemple comme stick de coiffage ou stick de maquillage des cheveux; comme composition de maquillage de la peau du visage ou du corps, ou des muqueuses, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; comme composition de soin de la peau ou des muqueuses, par exemple comme baume ou base de soin pour les lèvres, onguent pour le corps, crème de soin journalier; comme composition solaire ou auto-bronzante.

18. Composition selon l'une des revendications précédentes, se présentant sous la forme d'une composition "sans transfert" ou "non migrante" éventuellement colorée.

19. Composition selon l'une des revendications précédentes, se présentant sous la forme d'une composition de maquillage ou de soin non transfert, notamment comme rouge à lèvre non transfert ou fond de teint non transfert.

20. Procédé de traitement cosmétique d'un support choisi parmi la peau du visage ou du corps, les muqueuses et les fibres kératiniques, comprenant l'application sur ledit support d'une composition telle que définie dans l'une des revendications précédentes.

21. Utilisation, dans une composition cosmétique ou dermatologique se présentant sous forme solide et comprenant au moins une huile, d'une quantité suffisante d'au moins un composé de formule (I) pour structurer/gélifier ladite composition.

22. Composé de formule (I): 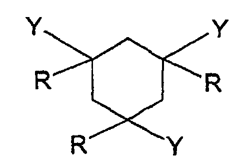 dans laquelle :
* R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
* Y représente un groupement choisi parmi les groupements suivants : -CO-S-R'; -CO-NHR'; -NH-COR' et -S-COR'; dans lesquels R' représente, indépendamment les uns des autres :
- un atome d'hydrogène,
- un groupement aryle, éventuellement substitué par une chaîne hydrocarbonée saturée ou insaturée, linéaire ou ramifiée, comprenant 1 à 22 atomes de carbone, notamment 10-18 atomes de carbone; ou
- une chaîne hydrocarbonée saturée ou insaturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy;
à la condition qu'au moins un desdits radicaux R' comprenne au moins une chaîne hydrocarbonée insaturée.

## Patentansprüche

1. Zusammensetzung und insbesondere kosmetische oder dermatologische Zusammensetzung, die in fester Form vorliegen kann und die mindestens eine Verbindung der folgenden Formel (I) enthält; 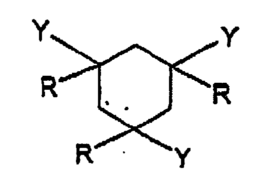 worin:
■ die Gruppen R unabhängig voneinander Wasserstoff oder eine geradkettige oder verzweigte, gesättigte Kohlenwasserstoffgruppe mit 1 bis 6 Kohlenstoffatomen und insbesondere 1 bis 4 Kohlenstoffatomen bedeuten; und
■ die Gruppen Y unter den folgenden Gruppen ausgewählt sind: -CO-S-R'; -CO-NHR'; -NH-COR' und -S-COR', wobei die Gruppen R' unabhängig voneinander bedeuten:
- Wasserstoff,
- eine Arylgruppe, die gegebenenfalls mit einer gesättigten oder ungesättigten, geradkettigen oder verzweigten Kohlenwasserstoffgruppe mit 1 bis 22 Kohlenstoffatomen und insbesondere 10 bis 18 Kohlenstoffatomen substituiert ist; oder
- eine gesättigte oder ungesättigte, geradkettige, verzweigte oder cyclische Kohlenwasserstoffgruppe mit 1 bis 22 Kohlenstoffatomen und insbesondere 10 bis 18 Kohlenstoffatomen, die gegebenenfalls mit einer oder mehreren Gruppen substituiert ist, die unter den Gruppen Aryl, Ester, Amid und Urethan ausgewählt sind; und/oder die gegebenenfalls ein oder mehrere Heteroatome enthält, die unter O, S und N ausgewählt sind; und/oder die gegebenenfalls mit einem oder mehreren Fluoratomen und/oder einer oder mehreren Hydroxygruppen substituiert ist;
mit der Maßgabe, daß mindestens eine Gruppe R' mindestens eine ungesättigte Kohlenwasserstoffgruppe enthält.

2. Zusammensetzung nach Anspruch 1, wobei mindestens eine der Gruppen R', vorzugsweise mindestens zwei Gruppen R' und noch besser alle drei Gruppen R' der Verbindung der Formel (I) eine geradkettige oder verzweigte Kohlenwasserstoffgruppe mit nur einer Doppelbindung bedeuten, die 1 bis 22 Kohlenstoffatome und insbesondere 10 bis 18 Kohlenstoffatome aufweist und gegebenenfalls mit einer oder mehreren Gruppen substituiert ist, die unter den Gruppen Aryl, Ester, Amid und Urethan ausgewählt sind; und/oder gegebenenfalls ein oder mehrere Heteroatome enthält, die unter O, S und N ausgewählt sind; und/oder gegebenenfalls mit einem oder mehreren Fluoratomen und/oder einer oder mehreren Hydroxygruppen substituiert ist.

3. Zusammensetzung nach einem der vorhergehenden Ansprüche, wobei die Gruppen R' der Verbindung der Formel (I) eine geradkettige oder verzweigte Kohlenwasserstoffgruppe mit nur einer Doppelbindung bedeuten, die 1 bis 22 Kohlenstoffatome und insbesondere 10 bis 18 Kohlenstoffatome aufweist.

4. Zusammensetzung nach einem der vorhergehenden Ansprüche, wobei die Gruppen R' der Verbindung der Formel (I) unter den Gruppen Caproleyl, Lauroleyl, Myristoleyl, Palmitoleyl, Oleyl, Gadoleyl, Linoleyl, Linolenyl und Elaidyl ausgewählt sind.

5. Zusammensetzung nach einem der vorhergehenden Ansprüche, wobei in der Form (I):
- R Wasserstoff oder Methyl bedeutet und/oder
- Y eine Gruppe -CO-NHR' oder -NH-COR' ist.

6. Zusammensetzung nach einem der vorhergehenden Ansprüche, wobei die Verbindung der Formel (I) ausgewählt ist unter:
- cis-1,3,5-Tris(oleylaminocarbonyl)cyclohexan,
- cis-1,3,5-Tris(palmitoylaminocarbonyl)cyclohexan,
- cis-1,3,5-Tris(lauroylaminocarbonyl)cyclohexan,
- cis-1,3,5-Tris(gadoleylaminocarbonyl)cyclohexan,
- cis-1,3,5-Tris(elaidylaminocarbonyl)cyclohexan,
- cis-1,3-Bis(oleylaminocarbonyl)-cis-5-(octadecylaminocarbonyl)cyclohexan,
- cis-1,3-Bis(oleylaminocarbonyl)-cis-5-(dodecylaminocarbonyl)cyclohexan,
- cis-1,3-Bis(oleylaminocarbonyl)-cis-5-[N-(3,7-dimethyloctyl)aminocarbonyl]cyclohexan,
- cis-1-(Oleylaminocarbonyl)-cis-3,5-bis(octadecylaminocarbonyl)cyclohexan,
- cis-1-(Oleylaminocarbonyl)-cis-3,5-bis(dodecylaminocarbonyl)cyclohexan,
- cis-1-(Oleylaminocarbonyl)-cis-3,5-bis[N-(3,7-dimethyloctyl)aminocarbonyl]cyclohexan,
- trans-1,3,5-Trimethyl-1,3,5-tris(oleylaminocarbonyl)cyclohexan, und
- trans-1,3,5-Trimethyl-1,3,5-tris(gadoleylaminocarbonyl)cyclohexan.

7. Zusammensetzung nach einem der vorhergehenden Ansprüche, wobei die Verbindung der Formel (I) in einer Menge von 1 bis 40 Gew.-%, beispielsweise 2 bis 10 Gew.-%, bezogen auf das Gesamtgewicht der Zusammensetzung, noch besser 3 bis 8 Gew.-% und sogar 4 bis 6 Gew.-% vorliegt.

8. Zusammensetzung nach einem der vorhergehenden Ansprüche, die im übrigen mindestens ein kosmetisch oder dermatologisch akzeptables Öl enthält.

9. Zusammensetzung nach einem der vorhergehenden Ansprüche, die mindestens ein Öl enthält, das unter den Kohlenwasserstoffölen und/oder Siliconölen und/oder fluorierten Ölen tierischen, pflanzlichen, mineralischen oder synthetischen Ursprungs ausgewählt ist, die flüchtig oder nicht flüchtig sind.

10. Zusammensetzung nach einem der vorhergehenden Ansprüche, die weniger als etwa 5 Gew.-% Wachs, bezogen auf das Gesamtgewicht der Zusammensetzung, insbesondere weniger als 2 Gew.-% und sogar weniger als 0,5 Gew.-% Wachs und vorzugsweise überhaupt keine Wachse (d.h. 0 %) enthält.

11. Zusammensetzung nach einem der vorhergehenden Ansprüche, die in fester Form vorliegt.

12. Zusammensetzung nach einem der vorhergehenden Ansprüche, die eine Härte im Bereich von 0,04 bis 3 N, vorzugsweise 0,1 bis 2,5 N und insbesondere 0,5 bis 2 N aufweist.

13. Zusammensetzung nach einem der vorhergehenden Ansprüche, die in durchscheinender oder sogar transparenter Form vorliegt.

14. Zusammensetzung nach einem der vorhergehenden Ansprüche, die durch eine Probe von 1 cm Dicke hindurch im Bereich von 400 bis 800 nm unabhängig von der Wellenlänge einen Wert der maximalen Lichtdurchlässigkeit von mindestens 2 % aufweist.

15. Zusammensetzung nach einem der vorhergehenden Ansprüche, die in Form einer Zusammensetzung vorliegt, die auf die Haut des Gesichts und des Körpers, auf die Schleimhäute und/oder auf Keratinsubstanzen, wie Nägel, Wimpern oder Haare, aufgetragen werden soll.

16. Zusammensetzung nach einem der vorhergehenden Ansprüche, die in Form eines festen oder weichen öligen Gels, das gegebenenfalls Wasser enthält; als feste Emulsion oder Emulsion in Gelform, Öl-in-Wasser-Emulsion, Wasser-in-Öl-Emulsion oder multiple Emulsion; Dispersion von Öl in Wasser; Mehrphasensystern und insbesondere Zweiphasensystem; Creme, Pomade, weiche Paste, Salbe, gegossener oder geformter Feststoff und insbesondere als Stift; transparentes wasserfreies festes Gel und genauer in Form eines wasserfreien durchscheinenden oder transparenten Stiftes vorliegt.

17. Zusammensetzung nach einem der vorhergehenden Ansprüche, die in Form einer Zusammensetzung zur Körperpflege, beispielsweise als Deostift; als Zusammensetzung für das Haar, beispielsweise als Stift zum Frisieren oder zum Schminken der Haare; Zusammensetzung zum Schminken der Haut des Gesichts oder des Körpers oder der Schleimhäute, beispielsweise als Lippenstift, Make-up in Stiftform oder in Tiegelchen gegossen, Wangenrouge, Lidschatten, fixierende Grundmasse, die auf einen herkömmlichen Lippenstift aufgetragen wird, Stift gegen Augenringe, Lipgloss, Eyeliner, Mascara, nicht permanentes Produkt zur Tätowierung; Zusammensetzung zur Pflege der Haut oder der Schleimhäute, beispielsweise als Lippenbalsam oder Pflegebasis für die Lippen, Salbe für den Körper, Tagescreme zur Pflege; Sonnenschutzmittel oder Selbstbräunungsmittel vorliegt.

18. Zusammensetzung nach einem der vorhergehenden Ansprüche, die in Form einer Zusammensetzung "ohne Transfer" oder "ohne Migration", die gegebenenfalls farbig ist, vorliegt.

19. Zusammensetzung nach einem der vorhergehenden Ansprüche, die in Form einer Zusammensetzung zum Schminken oder zur Pflege ohne Transfer und insbesondere als Lippenstift ohne Transfer oder Make-up ohne Transfer vorliegt.

20. Verfahren zur kosmetischen Behandlung eines Trägers, der unter der Haut des Gesichts oder des Körpers, den Schleimhäuten und den Keratinsubstanzen ausgewählt ist, das umfaßt, auf den Träger eine Zusammensetzung nach einem der vorhergehenden Ansprüche aufzubringen.

21. Verwendung mindestens einer Verbindung der Formel (I) in einer kosmetischen oder dermatologischen Zusammensetzung, die in fester Form vorliegt und mindestens ein Öl enthält, in einer Menge, die ausreichend ist, um die Zusammensetzung zu strukturieren/zu gelieren.

22. Verbindung der Formel (I): 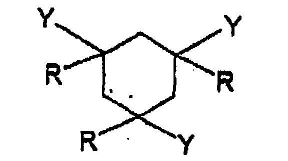 worin:
■ die Gruppen R unabhängig voneinander Wasserstoff oder eine geradkettige oder verzweigte, gesättigte Kohlenwasserstoffgruppe mit 1 bis 6 Kohlenstoffatomen und insbesondere 1 bis 4 Kohlenstoffatomen bedeuten; und
■ die Gruppen Y unter den folgenden Gruppen ausgewählt sind, -CO-S-R'; -CO-NHR'; -NH-COR' und -S-COR', wobei die Gruppen R' unabhängig voneinander bedeuten:
- Wasserstoff,
- eine Arylgruppe, die gegebenenfalls mit einer gesättigten oder ungesättigten, geradkettigen oder verzweigten Kohlenwasserstoffgruppe mit 1 bis 22 Kohlenstoffatomen und insbesondere 10 bis 18 Kohlenstoffatomen substituiert ist; oder
- eine gesättigte oder ungesättigte, geradkettige, verzweigte oder cyclische Kohlenwasserstoffgruppe mit 1 bis 22 Kohlenstoffatomen und insbesondere 10 bis 18 Kohlenstoffatomen, die gegebenenfalls mit einer oder mehreren Gruppen substituiert ist, die unter den Gruppen Aryl, Ester, Amid und Urethan ausgewählt sind; und/oder die gegebenenfalls ein oder mehrere Heteroatome enthält, die unter O, S und N ausgewählt sind; und/oder die gegebenenfalls mit einem oder mehreren Fluoratomen und/oder einer oder mehreren Hydroxygruppen substituiert ist;
mit der Maßgabe, daß mindestens eine Gruppe R' mindestens eine ungesättigte Kohlenwasserstoffgruppe enthält,

## Claims

1. Composition, in particular a cosmetic or dermatological composition, which may be in solid form, comprising at least one compound defined by formula (I) below: 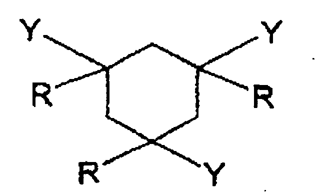 in which:
* R represents, independently of each other, a hydrogen atom or a linear or branched, saturated hydrocarbon-based chain containing 1 to 6 carbon atoms, in particular 1 to 4 carbon atoms;
* Y represents a group chosen from the following groups: -CO-S-R'; -CO-NHR'; -NH-COR' and -S-COR'; in which R' represents, independently of each other:
- a hydrogen atom,
- an aryl group, optionally substituted with a linear or branched, saturated or unsaturated hydrocarbon-based chain containing 1 to 22 carbon atoms, in particular 10-18 carbon atoms; or
- a linear, branched or cyclic, saturated or unsaturated hydrocarbon-based chain containing 1 to 22 carbon atoms, in particular 10-18 carbon atoms, optionally substituted with one or more groups chosen from aryl, ester, amide and urethane groups; and/or optionally comprising one or more hetero atoms chosen from O, S and N; and/or optionally substituted with one or more fluorine atoms and/or hydroxyl radicals;
on condition that at least one of the said radicals R' comprises at least one unsaturated hydrocarbon-based chain.

2. Composition according to Claim 1, in which at least one of the radicals R', better still at least two and even better still the three radicals R', of the compound of formula (I), represent(s) a linear or branched hydrocarbon-based chain comprising only one double unsaturation, containing 1 to 22 carbon atoms, in particular 10-18 carbon atoms, optionally substituted with one or more groups chosen from aryl, ester, amide and urethane groups; and/or optionally comprising one or more hetero atoms chosen from O, S and N; and/or optionally substituted with one or more fluorine atoms and/or hydroxyl radicals.

3. Composition according to either of the preceding claims, in which the radicals R' of the compound of formula (I) represent, a linear or branched hydrocarbon-based chain comprising only one double unsaturation, containing 1 to 22 carbon atoms, in particular 10-18 carbon atoms.

4. Composition according to one of the preceding claims, in which the radicals R' of the compound of formula (I) are chosen from caproleyl, lauroleyl, myristoleyl, palmitoleyl, oleyl, gadoleyl, linoleyl, linolenyl and elaidyl radicals.

5. Composition according to one of the preceding claims, in which, in formula (I)
- R represents a hydxogen atom or a methyl radical, and/or
- Y represents a group -CO-NHR' or -NH-COR'.

6. Composition according to one of the preceding claims, in which the compound of formula (I) is chosen from:
- cis-1,3,5-tris(oleylaminocarbonyl)cyclohexane,
- cis-1,3,5-tris(palmitoylaminocarbonyl)cyclohexane,
- cis-1,3,5-tris(lauroylaminocarbonyl)cyclohexane,
- cis-1,3,5-tris(gadoleylaminocarbonyl)cyclohexane,
- cis-1,3,5-tris(elaidylaminocarbonyl)cyclohexane,
- cis-1,3-bis(oleylaminocarbonyl)-cis-5-(octadecylaminocarbonyl)cyclohexane,
- cis-1,3-bis(oleylaminocarbonyl)-cis-5-(dodecylaminocarbonyl)cyclohexane,
- cis-1,3-bis(oleylaminocarbonyl)-cis-5-[N-(3,7-dimethyloctyl)aminocarbonyl]cyclohexane,
- cis-1-(oleylaminocarbonyl)-cis-3,5-bis(octadecylaminocarbonyl)cyclohexane,
- cis-1-(oleylaminocarbonyl)-cis-3,5-bis(dodecylaminocarbonyl)cyclohexane,
- cis-1-(oleylaminocarbonyl)-cis-3,5-bis[N-(3,7-dimethyloctyl)aminocarbonyl]cyclohexane,
- trans-1,3,5-trimethyl-1,3,5-tris(oleylaminocarbonyl)cyclohexane, and
- trans-1,3,5-trimethyl-1,3,5-tris(gadoleylaminocarbonyl)cyclohexane.

7. Composition according to one of the preceding claims, in which the compound of formula (I) is present in an amount of between 1% and 40% by weight, for example 2-10% by weight, relative to the total weight of the composition, and better still 3-8% by weight, or even 4-6% by weight.

8. Composition according to one of the preceding claims, moreover comprising at least one cosmetically or dermatologically acceptable oil.

9. Composition according to one of the preceding claims, moreover comprising at least one oil chosen from hydrocarbon-based oils and/or silicone oils and/or fluoro oils, which may be volatile or non-volatile, of animal, plant, mineral or synthetic origin.

10. Composition according to one of the preceding claims, comprising less than about 5% by weight, relative to the total weight of the composition, of wax, in particular less than 2% by weight, or even less than 0.5% by weight, of wax, and preferably comprising no waxes (i.e. 0%).

11. Composition according to one of the preceding claims, which is in solid form.

12. Composition according to one of the preceding claims, having a hardness of between 0.04 N and 3 N, preferably between 0.1 N and 2.5 N, in particular between 0.5 N and 2 N.

13. Composition according to one of the preceding claims, which is in translucent or even transparent form.

14. Composition according to one of the preceding claims, having a maximum light transmittance value, irrespective of its wavelength, of between 400 nm and 800 nm, through a 1 cm thick sample, of at least 2%.

15. Composition according to one of the preceding claims, which is in the form of a composition intended to be applied to the skin of the face and of the body, to mucous membranes and/or to keratin fibres such as the nails, the eyelashes or the hair.

16. Composition according to one of the preceding claims, which is in the form of a solid or soft oily gel, optionally comprising water; a solid or gelled oil-in-water, water-in-oil or multiple emulsion; a dispersion of oil in water; a multiphase system, in particular a two-phase system; a cream, a salve, a soft paste, an ointment or a cast or moulded solid and, in particular, a stick; a transparent anhydrous rigid gel and more especially in the form of a translucent or transparent anhydrous stick.

17. Composition according to one of the preceding claims, which is in the form of a body hygiene composition, for example in the form of deodorant sticks; as a hair composition, for example as a styling stick or a make-up stick for the hair; as a make-up composition for the skin of the face or of the body, or for mucous membranes, for example as a lipstick, a foundation cast as a stick or a dish, a face powder, an eyeshadow, a fixing base to be applied over a conventional lipstick, a concealer stick, a lipgloss, an eyeliner, a mascara or temporary tattoo products; as a care composition for the skin or mucous membranes, for example such as a lipcare balm or base, an ointment for the body or a daily care cream; as an antisun or self-tanning composition.

18. Composition according to one of the preceding claims, which is in the form of an optionally coloured "transfer-resistant" or "non-migrating" composition.

19. Composition according to one of the preceding claims, which is in the form of a transfer-resistant make-up or care composition, in particular such as a transfer-resistant lipstick or transfer-resistant foundation.

20. Process for the cosmetic treatment of a support chosen from the skin of the face or of the body, mucous membranes and keratin fibres, comprising the application to the said support of a composition as defined in one of the preceding claims.

21. Use, in a cosmetic or dermatological composition which is in solid form and which comprises at least one oil, of a sufficient amount of at least one compound of formula (I) to structure/gel the said composition.

22. Compound of formula (I) : 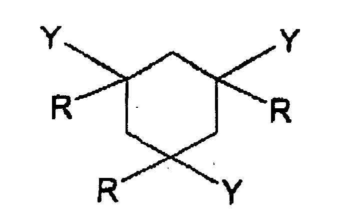 in which:
* R represents, independently of each other, a hydrogen atom or a linear or branched, saturated hydrocarbon-based chain containing 1 to 6 carbon atoms, in particular 1 to 4 carbon atoms;
* Y represents a group chosen from the following groups: -CO-S-R'; -CO-NHR'; NH-COR' and -S-COR'; in which R' represents, independently of each other:
- a hydrogen atom,
- an aryl group, optionally substituted with a linear or branched, saturated or unsaturated hydrocarbon-based chain containing 1 to 22 carbon atoms, in particular 10-18 carbon atoms; or
- a linear, branched or cyclic, saturated or unsaturated hydrocarbon-based chain containing 1 to 22 carbon atoms, in particular 10-18 carbon atoms, optionally substituted with one or more groups chosen from aryl, ester, amide and urethane groups; and/oroptionally comprising one or more hetero atoms chosen from O, S and N; and/or optionally substituted with one or more fluorine atoms and/or hydroxyl radicals;
on condition that at least one of the said radicals R' comprises at least one unsaturated hydrocarbon-based chain.
